# EUROPEAN PATENT APPLICATION

(11) **EP 4 094 785 A1**
(43) Date of publication of application: **30.11.2022**
(21) Application number: 21176600.1
(22) Date of filing: 28.05.2021
(51) Int. Cl.: A61M 1/16, A61M 1/28

(54) **INTEGRATED FILTER IN CASSETTE SYSTEM**

(71) Applicant: Bellco S.r.l., 41037 Mirandola (IT)
(72) Inventor: VESPERINI, Genni, 41037 Modena (IT); CAIAZZO, Marialuisa, 41037 Modena (IT); SICIGNANO, Luca, 41037 Modena (IT); TIMO, Alessia, 41037 Modena (IT); MARRA, Antonio Giuseppe, 41037 Modena (IT)
(74) Representative: Maschio, Antonio

(57) **Abstract**

A peritoneal dialysis system and cassettes for providing fluid connections and paths within the dialysis system are provided. The cassettes can include one or more filters and at least one pressure sensor to monitor the performance of the filters.

## Description

### FIELD

A peritoneal dialysis system and cassettes for providing fluid connections and paths within a dialysis system are provided. The peritoneal dialysis system can include one or more filters and at least one pressure sensor to monitor the performance of the filters. The filters and pressure sensors can be embedded in a cassette or positioned outside of the cassette. The cassette can be disposable.

### BACKGROUND

Peritoneal dialysis is a type of treatment for kidney failure that uses the lining of a patient's abdomen, the peritoneum, to filter blood while the blood remains inside the body. Peritoneal Dialysis is used to treat patients with End Stage Renal Disease and acute patients with renal failure. A catheter is provided for selective connection to a dialysis solution or a drain bag. A dialysis solution such as peritoneal dialysis fluid having water and electrolytes is warmed to a body temperature and then introduced into the peritoneal cavity of the patient via the catheter. Peritoneal dialysate is Class II drug and has certain sterility requirements. The peritoneal dialysate remains in the peritoneal cavity for a period of time required to clean the blood of waste products, toxins, and excess fluid leaving the correct amounts of electrolytes and nutrients in the blood. A peritoneal dialysis system or 'cycler' may repeat the exchange process a number of times (cycles), as required for a particular patient. However, systems that generate a peritoneal dialysis fluid from concentrates or pre-filled bags of fluids may fail to fully or adequately deliver a sterilize peritoneal dialysate to a patient. Moreover, systems and methods that use pre-packaged, pre-filled peritoneal dialysate can still lead to non-sterile fluid being delivered to a patient due to the introduction of pathogens of failure in fluid lines prior to delivering the fluid into a patient. As such, the known systems and methods that generate peritoneal dialysis from concentrates may start with sterile sources but nonetheless fail to deliver a sterile peritoneal dialysate to a patient.

Conventional peritoneal dialysis fluid includes purified water, glucose and electrolytes. The conventional systems require the use of multiple, pre-prepared bags of peritoneal dialysis fluid. The high weights and volumes of pre-filled and packaged peritoneal dialysis fluid bags result in the need for storing large, volumes and heavy supplies for each daily treatment (up to 15-18 Liters every day). Some systems attempt to avoid or minimize the use of pre-filled fluid bags by shipping peritoneal dialysis fluid components in the form of concentrates. However, the systems still require a large volume of liquid and multiple components to dissolve dry or liquid concentrates. The water or fluid used to dissolve the concentrates may not be sterile or does not meet the requirements for a peritoneal dialysate fluid. Even systems that ship peritoneal dialysate as pre-packaged sterile fluid or concentrates may experience contamination wherein the fluid is no longer sterile prior to being delivered to a patient. The conventional systems and methods can potentially introduce contamination along any point in the system prior to the peritoneal dialysate being delivered to a patient.

Hence, there is a need for systems and methods that can check a sterility of a peritoneal dialysate fluid. There is also a need for systems and methods for generating a sterile peritoneal dialysis fluid from concentrates and then checking the sterility of the generated fluid. The need includes sterilizing any fluid or generating a fluid into a state suitable for peritoneal dialysis prior to being delivered to a patient. The need still further includes systems and methods capable of removing bacteria and endotoxins from any fluid used to generate a peritoneal dialysis fluid. The need extends to systems and methods to ensure the proper functioning of the filters used to clean any fluid used to create a peritoneal dialysis fluid. The need includes cassettes that can generate a peritoneal dialysate fluid. The need still further includes a disposable cassette that can be discarded after use. The need also includes checking the sterility of any generated fluid including a peritoneal dialysate prepared from concentrates of any type including liquids, dry powders, slurries, or any combination thereof. The need further includes checking an online preparation and sterilization of peritoneal dialysis fluid. The need still further includes systems and methods for sensing a function or status of filters. The need includes checking and monitoring the functioning of microbial filters. The need includes positioning filters in a peritoneal dialysis system and at least one pressure sensor for monitoring filter performance. The need also includes sensing bacterial endotoxins present in a fluid. The need also includes sensing parameters that can lead to undesirable reactions. The need still further includes monitoring the sterility of a peritoneal dialysis fluid at any point during generation of the fluid and prior to delivery to a patient.

### SUMMARY OF THE INVENTION

The problem to be solved is sterilization of peritoneal dialysis fluid as well as systems and methods for proper functioning sterilization filters including microbial filters. The solution is to include filters in a peritoneal dialysis system and at least one pressure sensor for monitoring filter performance. The filters can optionally be positioned in a cycler.

The first aspect relates to a system. In any embodiment, the system can include a flow path fluidly connectable to a patient transfer set; at least one filter in the flow path; at least a first pressure sensor upstream of the filter to detect a fluid pressure on entry to the filter; and at least a second pressure sensor downstream of the filter to detect the fluid pressure on entry to and exit from the filter.

In any embodiment, the system can include a microprocessor programmed to collect a fluid pressure measurement from the first and second pressure sensors, the microprocessor further programmed to monitor changes in the fluid pressure on entry to and/or exit from the filter.

In any embodiment, the at least one filter and pressure sensors can be provided in the flow path upstream of a container of peritoneal dialysis fluid.

In any embodiment, the at least one filter can include at least a first filter and a second filter positioned in series.

In any embodiment, the system can have a pressure sensor downstream of the first filter, a pressure sensor upstream of the second filter and a pressure sensor between the first and second filters.

In any embodiment, the at least one filter, the first pressure sensor, and the second pressure sensor can be in a fluid line of a dialysis cassette.

In any embodiment, the at least one filter, the first pressure sensor, and the second pressure sensor can be embedded in the cassette.

In any embodiment, the disposable dialysis cassette can include a casing surrounding one or more fluid lines and at least one chamber open to one side covered by a flexible plastic film forming at least one pumping region.

In any embodiment, the system can include a preparator and cycler; the preparator can have a water source fluidly connectable to a plurality of containers each with one or more solutes; each container being fluidly connectable to a mixing bag, the mixing bag fluidly connectable to the cycler, wherein the filter and pressure sensor are provided in at least one of the fluid paths between the water source and the containers, the containers and the mixing bag, and/or the mixing bag and the cycler.

In any embodiment, the at least one filter, the first pressure sensor, and the second pressure sensor can be provided in the fluid path between the containers and the mixing bag.

In any embodiment, the mixing can be a bag for peritoneal dialysis fluid.

In any embodiment, the at least one filter can be provided in the fluid path downstream of the mixing.

In any embodiment, the at least one filter can include a first filter and a second filter; wherein the first filter is provided in the flow path between the containers and the mixing bag, and wherein the second filter is provided in the flow path downstream of the mixing bag.

In any embodiment, the filter can be a hollow fiber filter.

The features disclosed as being part of the first aspect can be in the first aspect, either alone or in combination, or follow any arrangement or permutation of any one or more of the described elements. Similarly, any features disclosed as being part of the first aspect can be in a second aspect described below, either alone or in combination, or follow any arrangement or permutation of any one or more of the described elements.

The second aspect relates to a dialysis cassette. In any embodiment, the dialysis cassette can include fluid lines having connectors for attachment to fluid paths external to the cassette, the fluid lines having at least one pumping region and at least one valve region for controlling the flow of fluids through the fluid lines, the cassette further including at least one filter in a fluid line, at least a first pressure sensor upstream of the filter to measure fluid pressure on entry to the filter, and at least a second pressure sensor downstream of the filter to measure fluid pressure on exit from the filter.

In any embodiment, the at least one filter can include at least two filters in series.

In any embodiment, the first pressure sensor can be provided upstream of a first filter and the second pressure sensor provided downstream of a second filter and the system can include a third pressure sensor being provided between the first and second filters.

In any embodiment, the at least one filter, the first pressure sensor, and the second pressure sensor can be embedded in the cassette.

In any embodiment, the cassette can include a casing surrounding the fluid lines and at least one chamber open to one side that is covered by a flexible plastic film for forming the at least one pumping region.

In any embodiment, the at least one filter can be a hollow fiber filter.

The features disclosed as being part of the second aspect can be in the second aspect either alone or in combination, or follow any arrangement or permutation of any one or more of the described elements. Similarly, any features disclosed as being part of the second aspect can be in the first aspect either alone or in combination, or follow any arrangement or permutation of any one or more of the described elements.

### BRIEF DESCRIPTION OF THE DRAWINGS

FIG. 1 is a schematic diagram of a filter and pressure sensor arrangement according to one embodiment for use in a peritoneal dialysis machine.
FIG. 2 is a schematic diagram of an alternative embodiment of a filter and pressure sensor arrangement for use in a peritoneal dialysis machine.
FIG.'s 3A-4D are respectively rear, front, side perspective and side views of a dialysis cassette having a filter and pressure sensor arrangement embedded therein according to an embodiment.
FIG. 4 is a block diagram of an automated peritoneal dialysis preparator and cycler machine.
FIG. 5 is a cassette system with non-embedded filters.
FIG.'s 6A-B illustrate a flow path through redundant filters.
FIG. 7 illustrates a non-limiting embodiment of disposable components for a peritoneal dialysis system.
FIG. 8 is a flow chart showing a process of testing and using redundant filters of a peritoneal dialysis system.

### DETAILED DESCRIPTION

Unless defined otherwise, all technical and scientific terms used have the same meaning as commonly understood by one of ordinary skill in the art.

The articles "a" and "an" are used to refer to one to over one (i.e., to at least one) of the grammatical object of the article. For example, "an element" means one element or over one element.

The term "attachment" refers to a connection between two or more components or materials.

The term "bag" as used herein refers to a container, and preferably a flexible, collapsible and/or foldable container. Preferably a bag is a container for fluids, most preferably for liquids.

The term "casing" refers to the outer walls of a component.

The term "chamber" refers to a container or a portion of container that can hold fluid.

The term "changes" refers to a difference in values for a given parameter.

The term "collect," when referring to a microprocessor, refers to receiving data from any source.

The term "comprising" includes, but is not limited to, whatever follows the word "comprising." Use of the term indicates the listed elements are required or mandatory but that other elements are optional and may be present.

The term "connector" refers to any component or connection allowing fluid to move between fluid lines or components.

The term "consisting of' includes and is limited to whatever follows the phrase "consisting of." The phrase indicates the limited elements are required or mandatory and that no other elements may be present.

The term "consisting essentially of' includes whatever follows the term "consisting essentially of' and additional elements, structures, acts or features that do not affect the basic operation of the apparatus, structure or method described.

A "container" refers to any structure or component capable of holding a solid, fluid, or gas.

The term "control" or "controlling" refers to a component or set of components directing the action of a material, component, or system.

The term "covered" refers to a component, a t least a portion of which is under the covering material.

A "cycler" or "peritoneal dialysis cycler" is a component or set of components for movement of fluid into and out of the peritoneal cavity of a patient.

A "dialysis cassette" refers to a set of components physically connected that are used in peritoneal dialysis therapy.

The term "disposable" refers to a component or set of components intended for use a single time, or a set amount of times and then replaced. No set minimum of times is associated with the term. For example, an item that can be used once or two or more times can be considered disposable in any embodiment.

The term "downstream" refers to a fluid path towards or nearer the cycler, further from the water source.

The term "embedded" refers to a component at least partially contained within a material.

The term "entry" refers to a fluid or gas going into a component, conduit, or container.

The term "exit" refers to a fluid or gas leaving a component, conduit, or container.

A "filter" a device inhibiting passage of particles, microbes or fragments of microbes such as endotoxins in a fluid or solution while allowing the passage of the fluid or solution.

A "flexible plastic film" refers to a polymer layer that can be bent or stretched without breaking.

The term "fluid" can be any substance without a fixed shape that yields easily to external pressure such as a gas or a liquid. Specifically, the fluid can be water, preferably purified water, and can be water containing any solutes at any concentration. The fluid can be used as a solvent (to dissolve one or more solutes). The fluid can also be dialysate of any type including fresh, partially used, or spent. The fluid can also contain one or more dissolved gas. The fluid may be blood.

A "fluid line" is any conduit through which fluid can move.

The term "fluid flow path" or "flow path" refers to any set of conduits or components that allow for the movement of fluids or gases.

The term "fluidly connectable" refers to the ability to provide passage of fluid from one point to another point. The ability to provide such passage can be any mechanical connection, fastening, or forming between two points to permit the flow of fluid, gas, or combinations thereof. The two points can be within or between any one or more of compartments, modules, systems, components, and rechargers, all of any type. Most preferably the connections are provided by connectors, fluid passage is provided by tubes or pipes.

The term "fluidly connected" refers to a particular state or configuration of one or more components such that fluid can flow from one point to another point. The connection state can also include an optional unconnected state or configuration, such that the two points are disconnected from each other to discontinue flow. It will be further understood that the two "fluidly connectable" points, as defined above, can form a "fluidly connected" state. The two points can be within or between any one or more of compartments, modules, systems, components, and rechargers, all of any type.

The term "formed" or "formed of' as used herein refers to a material comprising any of the materials, elements, composited or compounds listed.

A "hollow fiber filter" is a component having a plurality of fibers through which a fluid travels, the fibers preventing particles having a diameter larger than the inner diameter of the fiber from passing through.

The term "measuring" or "to measure" can refer to determining any parameter or variable. The parameter or variable can relate to any state or value of a system, component, fluid, gas, or mixtures of one or more gases or fluids.

The term "mix" means to combine one or more substance so that the resulting mixture is not easily separated. Mixing, especially evenly spreading, of solute and solvent aids and speeds the process of dissolution. Mixing can be achieved by any method, including spraying, stirring, shaking or otherwise agitating. Mixing can also refer to a situation wherein the components of a mixture are more evenly distributed and not clumped together. Mixing may be achieved, for example, by speeding the mixing up. In the context of a solution, mixing results in a solution of the correct concentration because all or most of the solutes dissolve, rather than remaining as clumps or aggregated within the solvent.

The term "mixing bag" refers to any bag or container in which components of a peritoneal dialysis fluid can be mixed. In certain embodiments, the mixing bag can also serve as a peritoneal dialysis fluid storage container.

The term "monitor" refers to the detection of one or more patient or system parameters.

The term "open to one side" refers to a container or component having an inlet or outlet on a single face of the container or component.

A "patient" or "subject" can be a member of any animal species, preferably a mammalian species, optionally a human. The subject can be an apparently healthy individual, an individual suffering from a disease, or an individual being treated for a disease. In certain embodiments, the patient can be a human, sheep, goat, dog, cat, mouse or any other animal.

The term "patient transfer set" refers to a set of components, such as fluid lines or conduits, through which peritoneal dialysis fluid can be infused into or drained out of a patient. The patient transfer set may or may not be disposable. The patient transfer set can be sterile and incorporate connections to a dialysis machine.

"Peritoneal dialysis" is a therapy wherein a dialysate is infused into the peritoneal cavity, which serves as a natural dialyzer. In general, waste components diffuse from a patient's bloodstream across a peritoneal membrane into the dialysis solution via a concentration gradient. In general, excess fluid in the form of plasma water flows from a patient's bloodstream across a peritoneal membrane into the dialysis solution via an osmotic gradient. Once the infused peritoneal dialysis solution has captured sufficient amounts of the waste components the fluid is removed. This cycle can be repeated for several cycles each day or as needed.

"Peritoneal dialysis fluid" is a fluid solution to be used in peritoneal dialysis having specified parameters for purity and sterility and containing one or more solutes. Peritoneal dialysis fluid is different than the dialysate used in hemodialysis.

A "peritoneal dialysis system" is a set of components capable of performing at least one function for peritoneal dialysis therapy.

The term "preparator" refers to a set of components used to generate peritoneal dialysis fluid.

The term "pressure," or "fluid pressure" refers to a force exerted by a gas or liquid on the walls of a component, container, or conduit.

A "pressure sensor" is a device capable of determining a force exerted by a gas or liquid on the walls of a component, container, or conduit.

The term "processor" or "microprocessor" as used is a broad term and is to be given an ordinary and customary meaning to a person of ordinary skill in the art. The term refers without limitation to a computer system, state machine, processor, or the like designed to perform arithmetic or logic operations using logic circuitry that responds to and processes the basic instructions that drive a computer. In any embodiment of the first, second, third, and fourth aspect, the terms can include ROM ("read-only memory") and/or RAM ("random-access memory") associated therewith.

The term "programmed," when referring to a processor, can mean a series of instructions that cause a processor to perform certain steps.

A "pumping region" is any portion of a component having at least part of a fluid pumping system.

"Purified water" can be defined as water produced by distillation, deionization, reverse osmosis, or other suitable processes that remove impurities in water.

The term "series" refers to an arrangement of components wherein fluid moving in a flow path generally used in treatment passes through each of the components in the series.

The term "solute" as used herein refers to a substance dissolved in a solution. The solute may comprise one or more chemicals or compounds. Solutes as used herein, before being dissolved in a solvent, may exist as entirely or partially dried, gels, concentrated liquids, pellets or powders, or any combination of these forms.

The term "solution" as used herein refers to a liquid mixture in which a solute is uniformly distributed within a solvent. A "solution for dialysis" may refer to any solution which can be used in any form of dialysis. It is envisaged that the solutions described herein contain specific concentrations of solutes.

The term "surrounding" refers to a material enclosing at least a portion of a component.

A "valve region" refers to any portion of a component having at least one valve.

The term "water purification module" refers to any single component or system which can produce purified water (as defined above) from tap water or other water containing any type of impurity.

The term "water source" refers to any source from which potable or non-potable water can be obtained.

The term "upstream" refers to a fluid path towards or nearer to the water source, furthest from the cycler.

### Peritoneal Dialysis System

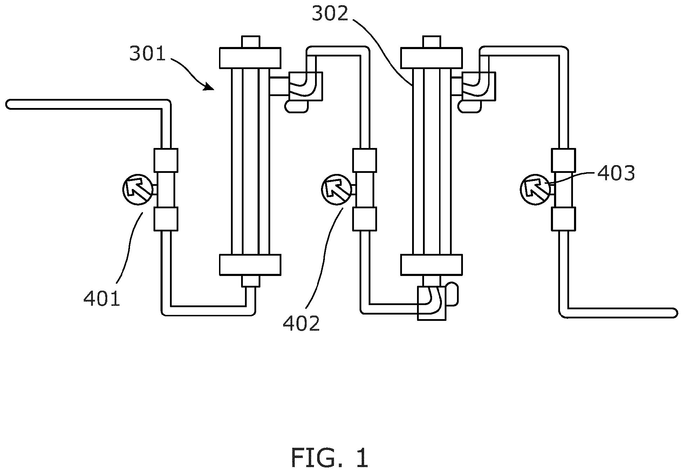

FIG. 1 shows a peritoneal dialysis system that can monitor and check a sterility of peritoneal dialysis fluid (PDF) to be delivered to a patient. FIG. 1 also shown an automated dialysis machine having a preparator and cycler for *in situ* preparation of peritoneal dialysis fluid (PDF) for delivery and drainage from a patient. In one embodiment, a PDF fluid line can include at least one filter. As illustrated in FIG. 1, the system can include a first filter **301** and a second filter **302** in series. However, any number of filters can be positioned in the system. For example, one filter can be used or three or more filters. Based on the filtering requirements and properties of the filter, one of ordinary skill can select and position an appropriate number and type of filters. One or more pressure sensors can be included for monitoring the performance of the first filter **301** and second filter **302.** For example, the system can include a first pressure sensor **401,** a second pressure sensor **402,** and a third pressure sensor **403,** one before the first filter **301,** one between the first filter **301** and second filter **302** and one after the second filter **302.**

The systems and methods can measure the pre-filter and post-filter pressure of the fluid path to detect overpressure or under-pressure. If a filter is blocked, the pressure can increase, and the filter break. Thus, the embodiment can provide a safety measure to check if a dialysis machine and related components including one or more filters are functioning correctly. In any embodiment, a pressure sensor **403** can also detect peritoneal pressure for the patient to provide faster pressure feedback, thereby enhancing the safety of the system for the patient. In any embodiment, the system and methods can use two filters to provide redundancy in compliance with ISO13408, increasing patient safety. The PDF should only be delivered to a patient if the fluid is sterile and non-pyrogenic.

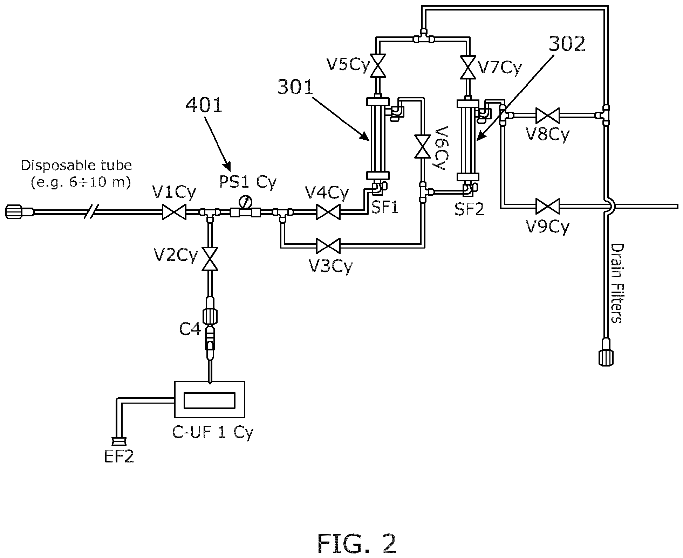

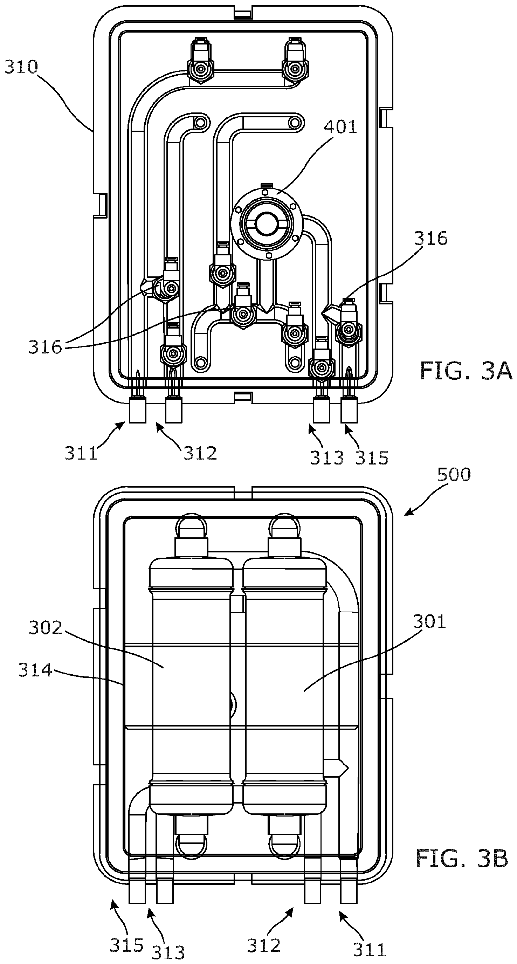

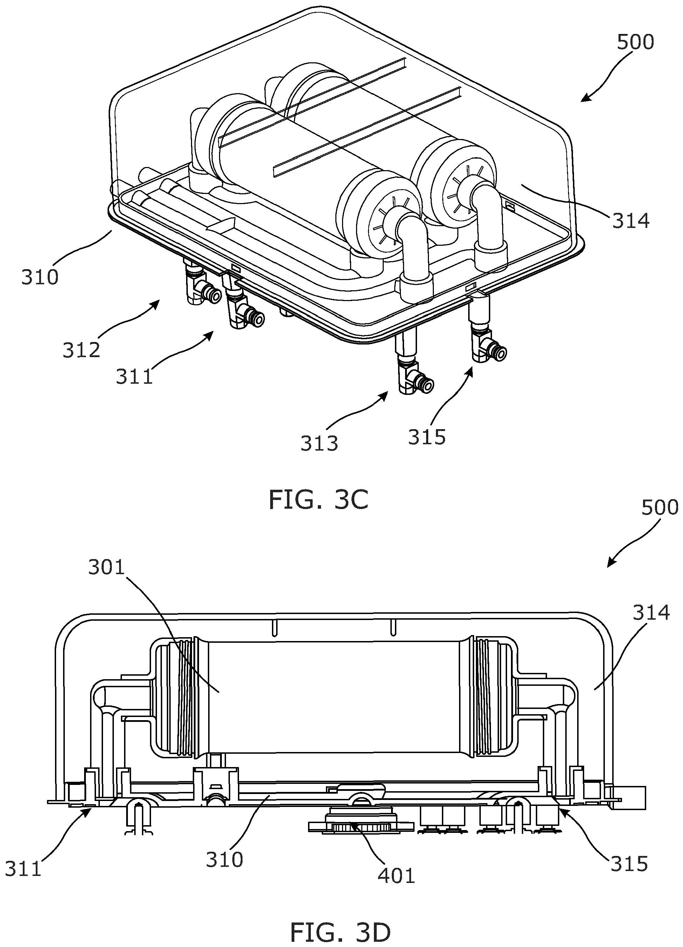

FIG. 2 illustrates an embodiment showing the filter circuit with an integrity test path. In FIG. 3, a single pressure sensor **401** can be provided upstream of the first filter **301** and a second filter **302.** In any embodiment, the integrity of each of filter **301** and filter **302** can be tested independently. To perform an integrity test, water from a water source (not shown) can be first flowed through filter **301** bypassing filter **302.** Next, valves v6 and v7 can be opened and water can be flowed through filter **302.** In the next step, valve v7 can be closed and v8 opened while water is flowed through both filters **301** and **302.** The process can then be repeated with air. In each step, the pressure decay rate through the filter can be measured by the pressure sensor **401.** One or more pressure can be used. If the pressure decay rate is not within acceptable limits, the filters can fail the integrity test. The filters, and optionally any other components can be replaced, and the test repeated until the system passes. In certain embodiments, the limits on pressure decay (ΔP/s) can vary from between 0.2 mmHg/s to 0.6 mmHg/s.

The pressure sensors can be in communication with one or more microprocessors. The microprocessors can be programmed to collect data transmitted by the pressure sensors, and to monitor changes in the fluid pressure on entry to and/or exit from the filter. The microprocessors can monitor the fluid pressure entering and exiting the filters during testing, PDF generation, and cycling of fluid to and from the patient. The pressure sensors and filters can be provided with or in a tubing system fluidly connectable to a dialysis machine. Alternatively, the pressure sensors and filters can be embedded within a dialysis cassette. The cassette can be used to selectively connect one or more fluid paths of the dialysis machine. The cassette can have mechanism for carrying out different actions on the fluid paths. For example, the cassette can include a pumping mechanism having at least one pump. The cassette can be configured to be inserted into a cassette holder of a dialysis machine to simplify set up and operation of the machine. The cassette can include a casing surrounding the fluid lines. In any embodiment, a dialysis cassette can include flexible valve domes in a valve region of the cassette. The cassette can also include flexible plastic film covered pumping chambers in a pumping region of the cassette that are actuated by mating pneumatic valves and pistons provided in the machine, thereby enabling fluid flow from fluid sources through the cycler and patient transfer set to a patient and to a drain to be controlled via the cassette. The pumping region can include a chamber that can be opened to one side that is covered in a flexible plastic film. The cassette can include connectors to attach tubing to the various fluid lines, such as the solution bags, patient line and drain. The cassette may also incorporate a heating region, for example in the form of a heating element embedded into a region of the cassette, to warm the fluid passing through the cassette. Alternatively, the fluid may be transported to a heating bag outside the cassette prior to delivery to the patient. In any embodiment, the cassette can be disposable. The disposable cassette can be constructed from lightweight materials. The disposable cassette can be fabricated at low cost. The disposable cassette can be recycled. The disposable cassette can be made from inexpensive polymers and plastics. The disposable cassette can be used one or more times prior to being exhausted.

FIG.'s 3A to 3D illustrate a non-limiting embodiment of a dialysis cassette **500.** FIG. 3A shows a rear or base **310** of the cassette **500** with fluid paths having connector **311** for connection to a drain line (not shown) and connector **312** for connection to a PDF line (not shown). Additional connector **313** and connector **315** can be provided for attachment of the components of the dialysis cassette **500** to additional fluid lines external to the dialysis cassette **500,** allowing bypass of the filters or additional flow paths. FIG. 3B shows a cassette **500** having a first filter **301** and a second filter **302** with a transparent view through a lid **314.** In FIG. 3B, the first filter **301** and the second filter **302** can be connected to one or more fluid paths and encased in any suitable housing such as a capped cylindrical housing. The lid **314** can be attached to the base **310** of the dialysis cassette **500** using any suitable mechanism to encase one or more interior components, including filter **301** and filter **302.** One or more valves **316** can be provided throughout the fluid paths to control fluid flow therethrough. The one or more valves **316** can be a pinch valve or any other suitable mechanism for occluding or directing flow in a flow path. Additionally, a first pressure sensor **401** can be positioned in any flow path of the dialysis cassette **500.** The lid **314** can be attached to the base **310** (not shown in FIG. 3B) of the cassette **500** to surround the interior components, including filter **301** and filter **302.** A second pressure sensor (not shown) can be provided downstream of filter **301** and filter **302.** The cassette **500** can also be provided with appropriate joints and seals to provide a connection of the filter **301** and filter **302** within the fluid path.

FIG. 3C is a perspective view of the cassette **500,** showing the filter **301** embedded in the cassette **500** between the lid **314** and base **310.** FIG. 3D is a side view of the cassette **500.** A pressure sensor **401** can be integral to the cassette to measure fluid pressure on entry or exit to the filter **301.** At least a second pressure sensor (not shown) can be included to measure the fluid pressure upon entry to and upon exit from the filter **301** and filter **302.**

FIG. 4 is a high-level block diagram of a peritoneal dialysis system having a preparator and a cycler. The system delivers water from a water source, such as a tap **20,** through a water purification module **40** and into one or more containers, which can include a first container **50,** a second container **51,** and a third container **52.** One of skill in the art will understand that any number of containers can be included, each with different concentrates to create a peritoneal dialysis fluid. The concentrates can be any combination of powder, liquid, or slurries. For example, the first container **50** contain a powder, the second container **51,** a slurry, and a third container **52,** a liquid concentrate. The system can then pump the generated concentrates to a mixing bag (not shown) to create the peritoneal dialysis fluid in a peritoneal dialysate fluid block being a preparator **60.** The water purification module **40** can include one or more of sorbent cartridges, carbon filters, reverse osmosis modules, and UV lights to remove chemical and some biological contaminants from the source water. One of ordinary skill will understand that other known water purification systems can be used. One of ordinary skill will understand that water purification is distinct from generating sterile water. The water from a source such as tap **20** can be flowed through the water purification module **40.** The resulting purified water can then be flowed into the first container **50,** second container **51,** and third container **52,** in any order or sequence.

Each of the first container **50,** second container **51,** and third container **52** can initially contain a constituent to create concentrate solution. The concentrates can be any combination of powder, liquid, or slurries, as described herein. The solutions can then be pumped to a cycler **100** connected to a patient transfer set for infusion into a patient **200.** The concentrates may be provided in any of constituent container **50,** container **51,** and container **52,** with each container having an inlet and outlet with a two-way valve whereby purified water can enter each container to dissolve a concentrate such as a powder. The resulting concentrates can then be delivered to a mixing bag prior to being pumped to the cycler **100.** The constituent container **50,** container **51,** and container **52** can also contain a concentrate liquid or slurry of partially dissolved solids and be connected to a degasser (not shown).

The cycler **100** can deliver fresh PDF to the patient **200** and drain used PDF from the patient **200** to a drain. In any embodiment, the described system can generate PDF from potable, tap, or drinking water of any type in combination with containers having powders, concentrates, or slurries. In FIG. 4, a cassette having the filters as shown in FIG.'s 3A to 3D can be provided in either the cycler **100,** preparator **60,** or both components of the system. The filters as shown in FIG.'s 3A to 3D can be positioned in the cycler **100** in fluid connection with the described mixing bag. In any embodiment, the any one or more of the filters FIG.'s 3A to 3D can be positioned before the mixing bag or after the mixing bag on the cycler **100,** but prior to the patient **200.** Alternatively, one or more of the redundant filters of FIG.'s 3A to 3D can be placed before the mixing bag and another filter placed after the mixing bag. In this manner, the filters can filter a fluid before being mixed in the mixing bag, and after being mixed in the mixing bag. Moreover, the system can position the filters as shown in FIG.'s 3A to 3D just prior to being passed to a transfer set or just prior to being flowed to the patient **200** to minimize possible contamination and deliver a sterile fluid.

In any embodiment, the peritoneal dialysis system having the preparator **60** and the cycler **100** can reconstitute dry powders into concentrates and proportion the resulting concentrates to create a PDF suitable for delivery to the patient **200.** In any embodiment, each of container **50,** container **51,** and container **52** can be provided with components and means to improve dissolution of the dry powder into a concentrate of liquid solution. The system can generate different amounts of fluid flow The system can include a reconstitution portion where powders are dissolved and a proportioning system to proportion a correct amount of each concentrate to a mixing bag. The reconstitution portion of the system can include the container **50,** container **51,** and container **52** filled with powders such as dextrose, magnesium, calcium, lactate, bicarbonate, and sodium chloride. In any embodiment, the lactate can be provided in slurry form. The slurry can be a semiliquid mixture containing particles of dry powder suspended in water. One or more valves can be positioned at an entry point and exit point to control fluid movement of any one of the container **50,** container **51,** and container **52.** A heater may be provided to aid dissolution and raise the temperature of the resultant PDF.

The cassettes described can filter the PDF and provide pressure monitoring to verify performance of the filters. The cassettes can provide a compact way to enhance patient safety by directing and controlling mixing of fluids and solutions. One of skill in the art will understand that all parts of the system can be made leak proof so that fluids do not escape and to help maintain sterility and non-pyrogenic fluid flow paths. Any of the containers may be made of any material, including polymers and plastics. The containers may be rigid or flexible. In certain embodiments, a flexible, resistant, stretchable container can be used. The containers may be made of multi-layered materials or single layered materials.

In certain embodiment, any one or more of the containers can have a V-shape, funnel shape, or cone shape section, with an opening for a nozzle at the point of the V, funnel, or cone. Such shape may maximize mixing of the fluid spray entering the container to aid dissolution. However, other types of containers, such as a solid container can be included.

Examples of solutes may include electrolytes, powdered acids or bases, citric acid, sodium chloride, hydrogen carbonate, calcium and magnesium ions and salts, sodium, potassium, sodium bicarbonate and all bicarbonate salt forms, sodium lactate, and osmotic agents such as glucose, dextrose, polydextrose, polydextrin, and xylitol.

As described, the dialysis system can include a cassette having one or more filters and pressure sensors. The system can also include other components such as heating elements, controllers, sensors, and scales. The system can be used at a patient's home, at a hospital or clinic, or in any other environment.

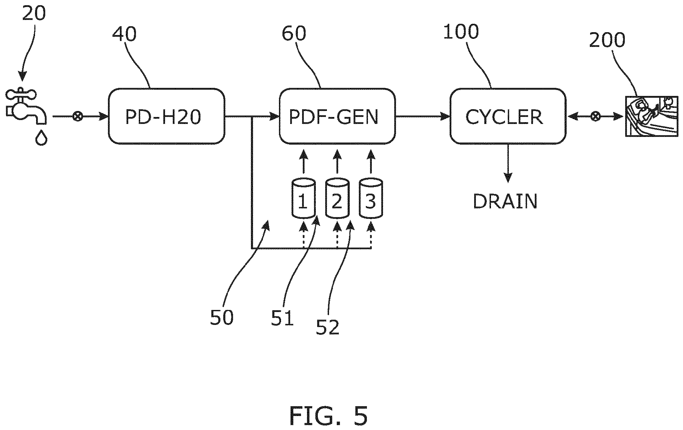

FIG. 5 illustrates a system having a first filter **301** and a second filter **302** outside of a cassette **300.** As described, the cassette **300** can include one or more fluid lines (not shown) for directing fluid through the peritoneal dialysis system. Fluid, such as one or more concentrates or generated peritoneal dialysis fluid, can pass through a fluid line of the cassette **300,** and into a fluid inlet **303** of the first filter **301,** as shown by line **304.** One or more degassers can be in fluid connection with any component of the system. The fluid can exit the first filter **301** through fluid outlet **306,** as shown by line **305,** and re-enter the cassette **300.** In a system using a second filter **302** in series with the first filter **301,** the fluid can enter through a fluid inlet (not shown) of second filter **302** and exit through fluid outlet **307** back into the cassette. Alternatively, fluid exiting the second filter **302** can be directed into a cycler, mixing bag, or any other component without re-entering the cassette **300.** Additional fluid outlet **308** on filter **301** and fluid outlet **309** on filter **302** can be included for removal of waste fluid. Outlet **308** and outlet **309** can be connected to a drain or waste container for disposal of the waste fluid.

One of skill in the art will understand that the fluid exiting filter **301** can directly enter filter **302,** and need not be flowed back through cassette **300.** Further, one or more additional fluid lines can be included connecting filter **301** and filter **302** to the cassette **300,** and the filters need not be directly attached to the cassette **300.** As described, pressure sensors (not shown in FIG. 5) can be included upstream and downstream of filter **301** and filter **302** to obtain a pre-filter and post-filter fluid pressure measurement. The pressure sensors can be embedded in the cassette **300** as illustrated in FIG.'s 3A-D, or can be positioned in fluid lines outside of the cassette **300.**

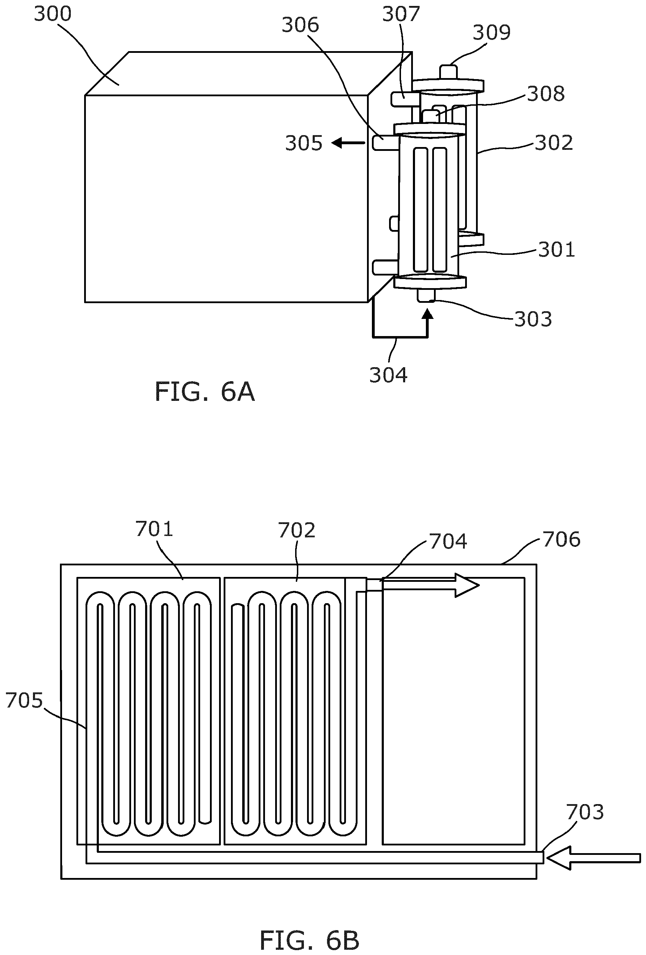

FIG's 6A-B show a fluid flow path **705** through a first filter **701** and second filter **702.** As illustrated in FIG. 6A, the first filter **701** and second filter **702** can be embedded within a cassette **706.** However, the first filter **701** and second filter **702** can be external to any cassette. Fluid enters first filter **701** through inlet **703.** The fluid follows flow path **705** into second filter **702,** before exiting through outlet **704.** In any embodiment, the filter can have two or more membranes. The membranes can have a flat shape to remove particles and microbiological contaminants. The flat membranes can be fully integrated with a cassette, as described. In any embodiment, a flat membrane can be used in the filters. In any embodiment, the membranes can have a pore size having rating to filter in particles in the range of 0.1 to 0.45 kDa. In certain embodiments, the membrane can have a pore size rating in a range from of 0.1 to 0.25 kDa. In other embodiments, the membrane can have a pore size equal to or less than 0.22 microns. One of ordinary skill can also select an appropriate pore size to provide a sterile fluid. One of ordinary skill can also select an appropriate pore size to remove unwanted particles and microbiological contaminants. In any embodiment, the membrane can possess an electrostatic charge. An electrostatic interaction can reduce contamination. The electrostatic charge can be provided by coating a membrane surface with cations using any technique known to those of skill in the art.

FIG. 6B is a cross section of the first filter **701** and second filter **702** in cassette **706.** The fluid passes through inlet **703** into flow path **705.** Fluid crosses first filter **701,** and then crosses through the second redundant filter **702** before exiting through outlet **704.** As described, pressure sensors (not shown) can be included both upstream and downstream of first filter **701** and second filter **702.** In certain embodiments, an additional pressure sensor can be included between first filter **701** and second filter **702.**

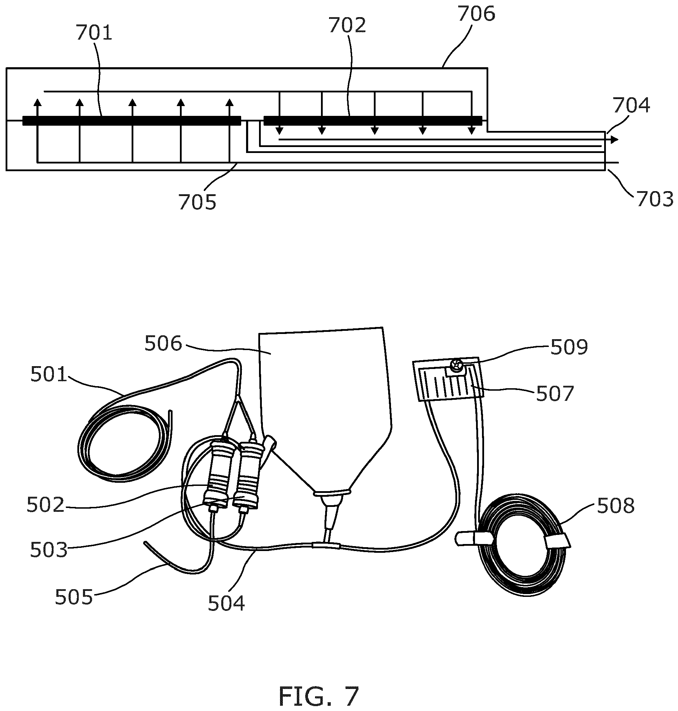

FIG. 7 shows a non-limiting embodiment of a system for peritoneal dialysis. In any embodiment, the cassettes used in the system can be discarded after one or more use. A first fluid line **501** can be connected to a preparator (not shown), as described. The preparator can include one or more containers for generating concentrates that are 3used to generate peritoneal dialysis fluid. The fluid line **501** can connect to a first filter **502** and second filter **503.** The first filter **502** and second filter **503** can remove endotoxins and bacteria from the source water and concentrates used by the preparator. Fluid exiting first filter **502** and second filter **503** can be flowed into mixing bag **506** through fluid line **504** where additional water can be added for mixing dilution of the concentrates to generate sterile peritoneal dialysis fluid. Waste fluid can exit first filter **502** and second filter **503** through drain line **505.** From the mixing bag **506,** the generated peritoneal dialysis fluid can pass through cassette **507** into fluid line **508** for connection to a patient or cycler. Pump **509** provides the driving force for moving fluid through the components of the system.

As described, in certain embodiments, first filter **502** and second filter **503** can be embedded in cassette **507.** Pressure sensors (not shown) can be included upstream and downstream, and optionally between first filter **502** and second filter **503.** Pump **509** can be embedded in cassette **507** as illustrated in FIG. 7, or can be located outside of the cassette **507.** Although shown as positioned between the preparator and mixing bag **506,** first filter **502** and second filter **503** can alternatively be positioned downstream of mixing bag **506.** Alternatively, first filter **502** can be positioned upstream of mixing bag **506** and second filter **503** can be positioned downstream of mixing bag **506.**

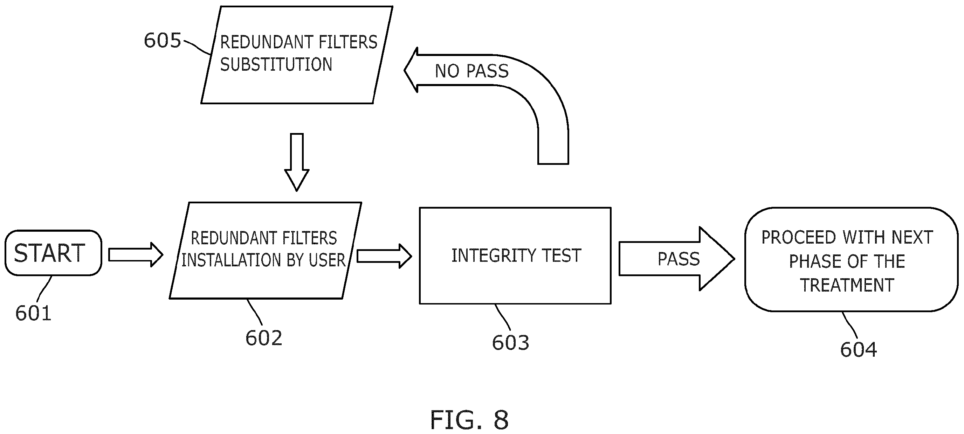

FIG. 8 is a flow chart showing the process of testing and using the redundant filters. The process can start in step **601.** In step **602,** the user can install the redundant filters. In step **603,** the system performs the described integrity test. If the filters pass the integrity test, the next phase of treatment can begin in step **604.** If the filters fail the integrity test, new filters can be substituted in step **605.**

In certain embodiments, the described filters can be hollow fiber filters. Hollow fiber filters can remove endotoxins and other contaminants from viscous fluids, such as concentrates and prepared peritoneal dialysis fluid, more efficiently than other types of filters, such as flat membrane filters. Hollow fiber filters can include a bundle of hollow fibers inside of a casing. The hollow fibers generally have an inner diameter of about 100µm to 1000µm packed at a specific density within a casing. One end of the hollow fibers can be placed in a potting material, which fills the interstitial void between the filters, thus preventing contaminated liquid from coming into contact with the filtrate.

By using hollow fiber filters, the filters can be included as part of the cycler, downstream of the preparator. To place the filters as part of the cycler, the filters can be operable to sterilize viscous fluids such as concentrates and generated peritoneal dialysis fluid at flow rates of between 0 ml/min to 600 ml/min. By including the filters as part of the cycler, the fluid can be sterilized just before infusion into a patient, eliminating possible contamination that can occur during or after formation, dilution, and mixing of the concentrates. Depending on the location of the filters on the cycler, connections downstream of the filters should be minimized or eliminated. Further, by placing the filters closer to the mixing bag, greater microbiological control is possible.

However, in certain embodiments, the filters can be included as part of the preparator, prior to generation of concentrates or peritoneal dialysis fluid. Including the filters as part of the preparator allows for a smaller cycler. Further, the filters can be integrated into the drain lines and use components, such as a compressor, already present in a preparator.

One skilled in the art will understand that various combinations and/or modifications and variations can be made in the described apparatus, methods and uses depending upon the specific needs for operation. Moreover, features illustrated or described as being part of an aspect may be used in the aspect either alone or in combination.

## Claims

1. A system, comprising:
a flow path fluidly connectable to a patient transfer set;
at least one filter in the flow path;
at least a first pressure sensor upstream of the filter to detect a fluid pressure on entry to the filter; and
at least a second pressure sensor downstream of the filter to detect the fluid pressure on entry to and exit from the filter.

2. The system of claim 1, further comprising a microprocessor programmed to collect a fluid pressure measurement from the first and second pressure sensors, the microprocessor further programmed to monitor changes in the fluid pressure on entry to and/or exit from the filter.

3. The system of claim 1, wherein the at least one filter and pressure sensors are provided in the flow path upstream of a container of peritoneal dialysis fluid.

4. The system of claim 1, wherein the at least one filter comprises at least a first filter and a second filter in series.

5. The system of claim 4, the system comprising a pressure sensor downstream of the first filter, a pressure sensor upstream of the second filter and a pressure sensor between the first and second filters.

6. The system of claim 1, wherein the at least one filter, the first pressure sensor, and the second pressure sensor are in a fluid line of a dialysis cassette.

7. The system of claim 6, wherein the at least one filter, the first pressure sensor, and the second pressure sensor are embedded in the dialysis cassette.

8. The system of claim 6, wherein the dialysis cassette comprises a casing surrounding one or more fluid lines and at least one chamber open to one side; the chamber covered by a flexible plastic film forming at least one pumping region.

9. The system of claim 1, further comprising a preparator and cycler;
the preparator comprising a water source fluidly connectable to a plurality of containers each with one or more solutes; each container fluidly connectable to a mixing bag , the mixing bag fluidly connectable to the cycler, wherein the filter and pressure sensor are provided in at least one of the fluid paths between the water source and the containers, the containers and the mixing bag, and/or the mixing bag and the cycler.

10. The system of claim 9, wherein the at least one filter, the first pressure sensor, and the second pressure sensor are provided in the fluid path between the containers and the mixing bag.

11. The system of claim 9, wherein the mixing bag is a bag for peritoneal dialysis fluid.

12. The system of claim 9, the at least one filter is provided in the fluid path downstream of the mixing bag.

13. The system of claim 9, wherein the at least one filter comprises a first filter and a second filter; wherein the first filter is provided in the flow path between the containers and the mixing bag, and wherein the second filter is provided in the flow path downstream of the mixing bag.

14. The system of claim 1, wherein the filter is a hollow fiber filter.

15. A dialysis cassette, comprising fluid lines having connectors for attachment to fluid paths external to the cassette, the fluid lines having at least one pumping region and at least one valve region for controlling the flow of fluids through the fluid lines, the cassette further comprising at least one filter in a fluid line, at least a first pressure sensor upstream of the filter to measure fluid pressure on entry to the filter, and at least a second pressure sensor downstream of the filter to measure fluid pressure on exit from the filter.

16. The disposable cassette of claim 15, wherein the at least one filter comprises at least two filters in series.

17. The disposable cassette of claim 16, the first pressure sensor upstream of a first filter, the second pressure sensor downstream of a second filter and further comprising a third pressure sensor between the first and second filters.

18. The disposable cassette of claim 15, wherein the at least one filter, the first pressure sensor, and the second pressure sensor are embedded in the cassette.

19. The disposable cassette of claim 15, wherein the cassette includes a casing surrounding the fluid lines and at least one chamber open to one side that is covered by a flexible plastic film for forming the at least one pumping region.

20. The disposable cassette of claim 15, wherein the filter is a hollow fiber filter.
